# EUROPÄISCHE PATENTANMELDUNG

(11) **EP 0 539 886 A1**
(43) Veröffentlichungstag der Anmeldung: **05.05.1993**
(21) Anmeldenummer: 92118190.5
(22) Anmeldetag: 23.10.1992
(51) Int. Cl.: A61K 31/70, A61K 31/715

(54) **Verwendung wässriger Kohlenhydrat-Lösungen als Spülflüssigkeit für die Arthroskopie**

(30) Priorität: 24.10.1991 DE 4135157
(71) Anmelder: LUITPOLD PHARMA GmbH, D-81379 München (DE)
(72) Erfinder: Gradinger, Reiner, Prof. Dr.-Med., W-8000 München 2 (DE); Träger, Jurka, Dr.-Med., W-8000 München 82 (DE); Klauser, Rainer, Dr. rer.nat., W-8000 München 60 (DE)
(74) Vertreter: Zumstein, Fritz, Dr.

(57) **Zusammenfassung**

Gegenstand der Erfindung ist die Verwendung von im wesentlichen ionenfreien wässrigen Lösungen von Kohlenhydraten als Spülflüssigkeit bei der Arthroskopie oder zur Gelenkspülung.

## Beschreibung

Die vorliegende Erfindung betrifft die Verwendung im wesentlichen ionenfreier wässriger Lösungen von Kohlenhydraten als Spülflüssigkeit bei der Arthroskopie oder zur Gelenkspülung.

Die Erfindung betrifft insbesondere den Einsatz der Lösungen von Monosacchariden, Lösungen von Oligosacchariden, Lösungen von Polysacchariden sowie Lösungsgemische aus Monosacchariden, Lösungsgemische aus Oligosacchariden, Lösungsgemische aus Polysacchariden, Lösungsgemische aus Monosacchariden und Polysacchariden sowie Lösungsgemische aus Oligosacchariden und Polysacchariden. Ein gemeinsames Merkmal der erfindungsgemäß eingesetzten Lösungen ist, daß sie im wesentlichen ionenfrei sind.

Der Begriff "im wesentlichen ionenfrei" bedeutet, daß die Gesamtkonzentration an gelösten Ionen im allgemeinen 20 mVal nicht überschreitet. Bevorzugt sind Lösungen, deren Gesamtkonzentration an gelösten Ionen kleiner ist als 5 mVal.

Die erfindungsgemäß eingesetzten Lösungen sind naturgemäß aufgrund ihres vorgesehenen Einsatzzweckes so beschaffen, daß sie zur Injektion geeignet sind. Insbesondere sind sie steril und pyrogenfrei.

Als Monosaccharide eignen sich insbesondere Aldosen, Ketosen und Zuckeralkohole. Hierbei kommen Monosaccharide mit 4, 5, 6 oder 7 Kohlenstoffatomen in Frage. Bevorzugt sind Aldohexosen, Ketohexosen und von ihnen abgeleitete Zuckeralkohole wie zum Beispiel Glucose, Galactose, Mannose, Fructose, Mannit, Dulcit und Sorbit. Besonders bevorzugt sind Glucose, Mannit und Sorbit.

Geeignete Oligosaccharide umfassen vorwiegend die Oligomeren der Glucose, der Mannose und der Galactose, bestehend aus bis zu sechs Monosaccharidbausteinen. Bevorzugt sind die Maltobiose, die Maltotriose, die Maltotetraose, die Cellobiose und die Cellotriose. Ferner umfassen die erfindungsgemäß eingesetzten Oligosaccharide solche Oligosaccharide, die aus verschiedenen Monosaccharidbausteinen aufgebaut sind, wie zum Beispiel die Lactose und die Saccharose.

Als Polysaccharide eignen sich natürlich vorkommende Polysaccharide, insbesondere Stärke und Dextrane sowie chemisch modifizierte Polysaccharide, insbesondere Hydroxyethylstärke oder Carboxymethylcellulose. Einer der charakteristischen Parameter für Polysaccharide ist das mittlere Molekulargewicht. Das mittlere Molekulargewicht der erfindungsgemäß eingesetzten Polysaccharide liegt insbesondere zwischen 5 000 und 100 000 Dalton, bevorzugt zwischen 30 000 und 80 000 Dalton.

Die Konzentration der Kohlenhydrate in den erfindungsgemäß eingesetzten Lösungen liegt insbesondere zwischen 0,1 - 20 Gew.%, bevorzugterweise zwischen 1 - 10 Gew.%. In einer besonders bevorzugten Ausführungsform der Erfindung enthalten die eingesetzten Lösungen die Kohlenhydrate in einer Gesamtkonzentration, die eine isotonische Lösung ergibt.

Der Begriff "isoton" wird durch den osmotischen Druck des Blutplasmas definiert und beträgt 290 mOsm/l.

Kohlenhydrathaltige wässrige Lösungen werden in der Medizin vielfach verwendet. Glucoselösungen dienen in erster Linie zur parenteralen Ernährung. Hypertone Lösungen von Mannit und Sorbit werden zur Auslösung einer Diurese und zur Bekämpfung von Ödemen verwendet. Schließlich dienen isotonische Kohlenhydratlösungen, insbesondere unter Beimischung hochmolekularer Polysaccharide wie Dextrane und Hydroxyethylstärke als Blutersatzflüssigkeit bei Operationen. Diese in der Medizin bisher gebräuchlichen Kohlenhydratlösungen enthalten vielfach weitere Substanzen wie zum Beispiel Salze und Aminosäuren. Durch den Zusatz von Salzen wird dabei das ionische Milieu der Körperflüssigkeiten imitiert. Der Zusatz verschiedener Salze wie NaCl, KCl oder NaHCO₃ dient auch der Isotonisierung, das heißt der Einstellung der Osmolarität der Lösung auf die Osmolarität des Blutplasmas.

Im Zusammenhang mit der vorliegenden Erfindung wurde nun festgestellt, daß sich im wesentlichen ionenfreie wässrige Lösungen von Kohlenhydraten als Spülflüssigkeit bei der Arthroskopie und zur Gelenkspülung besonders eignen.

Bei der Arthroskopie und zur Gelenkspülung werden als Spülflüssigkeiten bisher physiologisch verträgliche Flüssigkeiten wie destilliertes Wasser, physiologische (0,9 %) Kochsalzlösung, Ringer-Lösung oder Ringer-Laktat-Lösung verwendet. Ringer-Lösung enthält 8,6 g NaCl, 0,3 g KCl und 0,33 g CaCl₂ x 2 H₂O pro Liter. Ringer-Laktat-Lösung enthält 6 g NaCl, 0,4 g KCl, 0,27 g CaCl₂ x 2 H₂O und 3,05 g Natriumlaktat pro Liter. Die Verwendung dieser Lösungen als Spülflüssigkeit in der Arthroskopie ist in der folgenden Literaturstelle beschrieben: Arciero, R. A. et al., 1986, Orthopedics 9, 1511-1515. Ferner wurde eine chirurgische Irrigationsflüssigkeit beschrieben, die auch für endoskopische und arthroskopische Eingriffe geeignet ist (FR -A- 2 548 897).

Aus International Orthopaedics 10:101-104 (1986) ist es ferner bekannt, bei der Arthroskopie als Spülflüssigkeit eine Lösung von Dextran 40 000 in physiologischer Kochsalzlösung zu verwenden; durch die erhöhte Osmolarität soll die Entstehung eines Synovialödems verhindert werden.

Bei Spülflüssigkeiten für andere Zwecke ist ebenfalls die Verwendung von Kohlenhydraten in Salzlösungen beschrieben, nämlich von Dextran 40 000 für intraoculare Spülungen (US-PS 4 238 482) sowie von Mannit zur Spülung des Dickdarms (Dtsch. med. Wochenschrift 108 (51/52), 1983, S. 1959-1964). Auch dabei werden andere Zielsetzungen verfolgt und mit anderen Lösungen angestrebt als bei der vorliegenden Erfindung.

In Untersuchungen, die zur vorliegenden Erfindung führten, wurde gefunden, daß die bei der Spülung erfolgende, jedoch unerwünschte Herauslösung von Proteoglykanen aus dem Knorpel ein brauchbares Kriterium für die Eignung der Spülflüssigkeit ist. Proteoglykane sind ein Hauptbestandteil des Knorpels. Sie dienen als "Kitt" zwischen den Kollagenfasern und sind wegen ihrer physikochemischen Eigenschaften, insbesondere wegen des Wasserbindungsvermögens, für die biomechanischen Eigenschaften des Knorpels unerläßlich (Literatur: Handley, C. J. et al. (1985) The structure and synthesis of proteoglycans of articular cartilage. Cell Biol. Int. Reports 9, 753-782). Proteoglykane werden schon nach kurzem Kontakt mit der Spülflüssigkeit, nämlich im Verlauf von 1 bis 2 Stunden, aus dem Knorpel herausgelöst. Diese Einwirkungszeit entspricht der Dauer arthroskopischer Operationen. Das Herauslösen von Proteoglykan aus dem Knorpel ist aber unerwünscht, da der Verlust dieses Hauptbestandteils der Knorpelmatrix zur Verschlechterung der biophysikalischen Eigenschaften des Knorpels, insbesondere seiner Elastizität und des Wasserbindungsvemögens führt.

Wie im nachfolgenden gezeigt wird, zeigen alle bisher gebräuchlichen Spülflüssigkeiten den Nachteil des Herauslösens von Proteoglykan aus dem Knorpel. Aufgabe der Erfindung war es deshalb, Spülflüssigkeiten zu finden, die während des Kontaktes mit dem Knorpel möglichst wenig Proteoglykan aus dem Knorpel herauslösen.

Im Zusammenhang mit der vorliegenden Erfindung wurde nun überraschend festgestellt, daß wässrige Lösungen von Kohlenhydraten besonders wenig Proteoglykan aus dem Knorpel herauslösen. Diese vorteilhafte Eigenschaft kommt auch schon bei der kurzen Einwirkungszeit von ein bis zwei Stunden zum Tragen.

Operative und diagnostische Eingriffe mittels Arthroskopie beschränken sich im allgemeinen auf eine Zeitdauer von längstens 6 Stunden, in der Regel auf längstens 4 Stunden. Demgemäß ist die vorliegende Erfindung insbesondere auf eine Anwendungszeit der anspruchsgemäß verwendeten Lösungen von höchstens 6 Stunden, vorzugsweise höchstens 4 Stunden gerichtet.

Die besondere Eignung der erfindungsgemäß eingesetzten Lösungen von Kohlenhydraten als Spülflüssigkeit für die Arthroskopie läßt sich mit dem nachstehend beschriebenen Versuch zeigen.

### Versuch:

### Bestimmung des Proteoglykanverlustes bei Inkubation von Knorpel in verschiedenen wässrigen Lösungen

Gelenkknorpel vom Rind wurde aus dem Metakarpalgelenk frisch geschlachteter Stiere (Alter etwa 2 Jahre) gewonnen. Der Knorpel wurde mit dem Skalpell vom subchondralen Knochen abgetrennt, in kleine Stücke geschnitten und bis zur Einwaage in einem Reagenzglas verschlossen aufbewahrt.
Etwa 25 mg Knorpel wurden genau eingewogen und mit der zu untersuchenden Lösung in einer solchen Menge versetzt, daß ein Verhältnis von 1 ml Lösung zu 25 mg Knorpel resultierte. Anschließend wurde für 2 Stunden bei Raumtemperatur inkubiert. Die überstehende Lösung wurde abpipettiert, und der Gehalt der Lösung an Proteoglykan wurde mittels der Metachromasie von Dimethylmethylenblau nach der Methode von Carroll bestimmt (Carroll, G. J., 1987, Ann. Rheum. Dis. 46, 375-390). Als Referenzsubstanz diente Chondroitinsulfat A, mit dem eine Eichkurve erstellt wurde. Pro untersuchter Lösung wurde eine Dreifachbestimmung vorgenommen. Die Ergebnisse der Einzelbestimmungen wurden gemittelt. In der nachstehenden Tabelle sind die Mittelwerte der Proteoglykangehalte verschiedener Spülflüssigkeiten nach zweistündiger Inkubation mit Knorpel dargestellt. Hierbei ist der Proteoglykangehalt von destilliertem Wasser gleich 100 % gesetzt.

**Tabelle**

| Proteoglykangehalt verschiedener wässriger Lösungen nach zweistündiger Inkubation mit Knorpel | | | |
|---|---|---|---|
| Lösung | | Beispiel | Proteoglykangehalt (%) |
| Dest. Wasser | | Vergleich | 100 |
| 0,9 % NaCl | | Vergleich | 949 |
| Ringer-Lösung | | Vergleich | 354 |
| 2 % | Mannit | 1 | 65 |
| 10 % | Mannit | 2 | 21 |
| 5 % | Sorbit | 3 | 63 |
| 20 % | Sorbit | 4 | 38 |
| 5 % | Glucose | 5 | 77 |
| 10 % | Dextran 70 | 6 | 31 |
| 2 % | Dextran 70 + | 7 | 40 |
| 5 % | Sorbit | | |
| 10 % | Dextran 40 + | 8 | 15 |
| 5 % | Glucose | | |

Aus der Tabelle ist ersichtlich, daß die erfindungsgemäß eingesetzten Lösungen wesentlich weniger Proteoglykan herauslösen als die beste der Vergleichslösungen, nämlich dest. Wasser. Die erfindungsgemäß eingesetzten Kohlenhydratlösungen sind deshalb den herkömmlichen Spülflüssigkeiten überlegen, was nicht vorhersehbar war.

Folgende Beispiele erläutern die Erfindung, ohne sie darauf zu beschränken:

### Beispiel 1

Man löst 20 g Mannit in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 2

Man löst 100 g Mannit in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 3

Man löst 50 g Sorbit in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 4

Man löst 200 g Sorbit in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 5

Man löst 50 g Glucose in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 6

Man löst 100 g Dextran 70 in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 7

Man löst 50 g Sorbit und 20 g Dextran 70 in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 8

Man löst 50 g Glucose und 100 g Dextran 40 in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 9

Man löst 50 g Lactose in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

### Beispiel 10

Man löst 60 g Fructose in ca. 800 ml destilliertem Wasser, überführt in einen 1 l-Meßkolben und füllt bis zur Marke auf. Man filtriert über ein 0,2 µ Membranfilter steril und füllt in eine sterilisierte 1 l-Flasche ab.

## Patentansprüche

1. Verwendung von im wesentlichen ionenfreien wässrigen Lösungen von Kohlenhydraten als Spülflüssigkeit bei der Arthroskopie oder zur Gelenkspülung.

2. Verwendung von im wesentlichen ionenfreien wässrigen Lösungen von Monosacchariden gemäß Anspruch 1.

3. Verwendung von im wesentlichen ionenfreien wässrigen Lösungen von Oligosacchariden gemäß Anspruch 1.

4. Verwendung von im wesentlichen ionenfreien wässrigen Lösungen von Polysacchariden gemäß Anspruch 1.

5. Verwendung von im wesentlichen ionenfreien wässrigen Lösungen enthaltend Gemische aus Monosacchariden und Polysacchariden gemäß Anspruch 1.

6. Verwendung von im wesentlichen ionenfreien wässrigen Lösungen enthaltend Gemische von Monosacchariden gemäß Anspruch 1.

7. Verwendung von im wesentlichen ionenfreien wässrigen Lösungen enthaltend Gemische von Oligosacchariden gemäß Anspruch 1.

8. Verwendung von im wesentlichen ionenfreien wässrigen Lösungen enthaltend Gemische von Oligosacchariden und Polysacchariden gemäß Anspruch 1.

9. Verwendung von im wesentlichen ionenfreien wässrigen Lösungen enthaltend Gemische von Polysacchariden gemäß Anspruch 1.

10. Verwendung von Lösungen gemäß Anspruch 1, dadurch gekennzeichnet, daß die einzelnen Kohlenhydrate in einer Konzentration von 0,1 - 20 Gew.% enthalten sind.

11. Verwendung von Lösungen gemäß Anspruch 1, dadurch gekennzeichnet, daß die Kohlenhydrate in einer Gesamtkonzentration vorhanden sind, die eine isotonische Lösung ergibt.
